# EUROPÄISCHE PATENTSCHRIFT

(11) **EP 0 419 412 B1**
(45) Veröffentlichungstag und Bekanntmachung des Hinweises auf die Patenterteilung: **10.11.1993**
(21) Anmeldenummer: 90810687.5
(22) Anmeldetag: 11.09.1990
(51) Int. Cl.: C07D 249/20

(54) **Verfahren zur Herstellung von Benztriazolen**
Process for the preparation of benzotriazoles
Procédé pour la préparation de benzotriazoles

(30) Priorität: 20.09.1989 CH 3420/89
(43) Veröffentlichungstag der Anmeldung: 27.03.1991
(73) Patentinhaber: CIBA-GEIGY AG, 4002 Basel (CH)
(72) Erfinder: Prestel, Helmut, Dr., D-7520 Bruchsal (DE); Maul, Rudolf, Dr., D-6143 Lorsch/Hessen (DE)

(56) Entgegenhaltungen:
- EP-A- 0 380 839
- GB-A- 1 494 823

## Beschreibung

Die vorliegende Erfindung betrifft ein Verfahren zur Herstellung von 2-(2-Hydroxyphenyl)-2H-benztriazolen durch katalytische Hydrierung von o-Nitrophenylazohydroxyphenyl-Verbindungen in Gegenwart eines Edelmetall-Hydrierkatalysators und von bestimmten organischen Aminen.

2-(2-Hydroxyphenyl)-2H-benztriazole sind als wertvolle UV-Absorber aus der Literatur bekannt. Sie werden in der Praxis in grossem Umfang als Lichtstabilisatoren für eine Vielzahl von Substraten eingesetzt, beispielsweise zur Stabilisierung von thermoplastischen Kunststoffen und von Ueberzugsmaterialien (z.B. von Lacken), aber auch in diversen Aufzeichnungsmaterialien (z.B. in photographischen Schichten und Papieren sowie in Drucktinten und Druckpapieren) und in Textilien.

Entsprechend der Bedeutung dieser Verbindungen wurde bereits eine ausserordentlich grosse Anzahl von Verfahren zu deren Herstellung vorgeschlagen. Ein Grossteil davon geht von den oben genannten o-Nitrophenylazo-Verbindungen aus und bedient sich der reduktiven Cyclisierung nach verschiedenen Reduktionsverfahren. Eines dieser Reduktionsverfahren ist die katalytische Hydrierung, die für die genannten Benztriazole in einer Reihe von Publikationen beschrieben wurde.

Die US-A 3,978,074 beschreibt ein in alkalischem und vorzugsweise in wässrigem Medium durchgeführtes Hydrierverfahren der vorstehend erwähnten Art, wobei als Hydrierkatalysatoren die üblichen Edelmetall- und andere Metallkatalysatoren eingesetzt werden. Gemäss GB-A 1 494 825 und 1 494 824 erfolgt die Hydrierung ebenfalls in alkalischem rein wässrigem (GB-A 1 494 825) oder wässrig/organischem (GB-A 1 494 824) Medium. Als Hydrierkatalysatoren werden Edelmetalle eingesetzt. Das in der GB-A 1 494 823 beschriebene Hydrierverfahren wird in organischen Lösungsmitteln unter Verwendung von organischen Aminen als Basen und der üblichen Edelmetallkatalysatoren durchgeführt. Die US-A 4,219,480 lehrt die Verwendung eines Nickelkatalysators als Hydrierkatalysator.

Auch die JP-A 52-113 973 und 52-113 974 befassen sich mit der Herstellung von 2-(2-Hydroxyphenyl)-2H-benztriazolen durch katalytische Hydrierung. Als Katalysator wird unter anderem auch 5 % Platin auf Kohle eingesetzt. In der zweitgenannten japanischen Veröffentlichung sind auch Polyalkylpolyamine als Basen beschrieben, doch ist in allen Ausführungsbeispielen die Mitverwendung eines wasserinischbaren Lösungsmittels (eines Alkohols) vorgesehen. Die erzielten Ausbeuten sind relativ niedrig.

Es wurde nun überraschend gefunden, dass das erwähnte Reduktionsverfahren besonders günstig und wirtschaftlich ausgeführt werden kann, wenn man eine bestimmte Klasse von Aminen und bestimmte Lösungsmittel einsetzt.

Das erfindungsgemässe Verfahren zur Herstellung von 2-(2-Hydroxyphenyl)-2H-benztriazolen der Formel 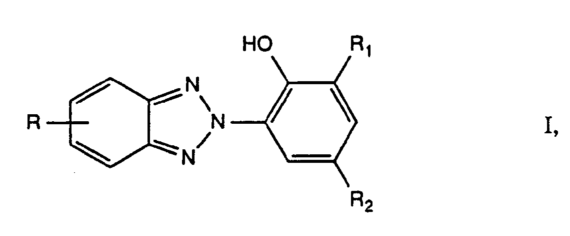
worin R Wasserstoff, C₁-C₁₂-Alkyl oder C₁-C₄-Alkoxy, R₁ Wasserstoff, C₁-C₁₂-Alkyl, C₅-C₆-Cycloalkyl, Phenyl oder Phenyl-C₁-C₄-alkyl und R₂ C₁-C₁₂-Alkyl, C₅-C₆-Cycloalkyl, Phenyl, Phenyl-C₁-C₄-alkyl oder eine Gruppe -CₙH₂ₙ-COOR₃ bedeuten, worin n für 0 bis 4 und R₃ für Wasserstoff oder C₁-C₁₂-Alkyl stehen, durch katalytische Hydrierung einer Azoverbindung der Formel 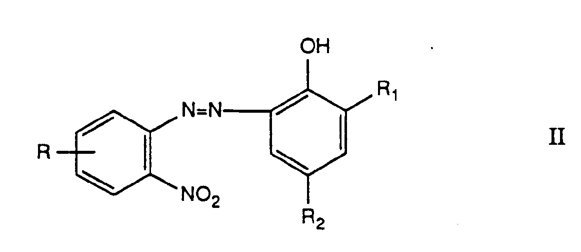
in Gegenwart eines Edelmetall-Hydrierkatalysators und eines organischen Amins ist dadurch gekennzeichnet, dass man als Hydrierkatalysator Pt, Pd, Pt/Pd oder Rh auf einem Träger und als Amin ein Alkylendiamin oder ein acyclisches oder cyclisches Polyalkylen-polyamin einsetzt, wobei die Stickstoffatome der genannten Amine unsubstituiert oder, unabhängig voneinander, mit C₁-C₆-Alkyl subststituiert sind, und die Hydrierung in einem aromatischen Kohlenwasserstoff oder halogenierten aromatischen Kohlenwasserstoff oder Mischungen der genannten Kohlenwasserstoffe mit Wasser als Lösungsmittel durchführt, wobei man die Hydrierung von Azoverbindungen der Formel II, worin R₂ = -CₙH₂ₙCOOH, in Wasser oder in Mischungen der genannten Kohlenwasserstoffe und Wasser durchführt.

In Formel I ist Phenyl-C₁-C₄-alkyl (R₁, R₂) vorzugsweise Benzyl, Phenethyl, α-Methylbenzyl und α,α-Dimethylbenzyl, insbesondere Benzyl oder α,α-Dimethylbenzyl. R₃ ist vorzugsweise H oder C₁-C₄-Alkyl, insbesondere H oder Methyl. Aus den nieder-Alkylestern, insbesondere dem Methylester, können z.B. durch nachträgliche Verseifung Verbindungen mit R₃ = H oder durch Umesterung Verbindungen mit anderen Alkylgruppen R₃ hergestellt werden. Als Cycloalkyl ist insbesondere Cyclohexyl zu erwähnen. Wenn R, R₁, R₂ oder R₃ Alkylgruppen bedeuten, haben diese vorzugsweise 1 bis 8, insbesondere 1-5 C-Atome.

Die Ausgangsverbindungen der Formel II sind bekannt, z.B. aus den eingangs angeführten Druckschriften oder aus der EP-A 57 160, oder sie können nach den dort angegebenen Verfahren hergestellt werden, etwa durch Diazotierung eines o-Nitroanilins der Formel 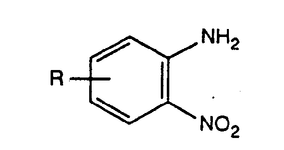
und Kupplung des resultierenden Diazoniumsalzes mit einem Phenol der Formel

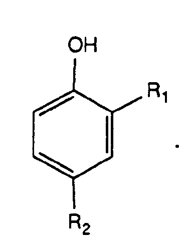

Bevorzugt werden Verbindungen der Formel I hergestellt, worin R Wasserstoff oder C₁-C₁₂-Alkyl, insbesondere Wasserstoff ist, z.B. solche Verbindungen der Formel I, worin R₁ Wasserstoff, C₁-C₁₂-Alkyl oder Phenyl-C₁-C₃-alkyl (insbesondere α,α-Dimethylbenzyl) und R₂ C₁-C₁₂-Alkyl (z.B. C₁-C₈-Alkyl), Phenyl-C₁-C₃-alkyl (insbesondere α,α-Dimethylbenzyl) oder eine Gruppe -C₂H₄COOR₃ bedeuten, worin R₃ H oder C₁-C₁₂-Alkyl (z.B. C₁-C₈-Alkyl), insbesondere H oder C₁-C₄-Alkyl, bedeutet.

Von besonderer praktischer Bedeutung ist die Herstellung von Verbindungen der Formel I, worin R₁ Wasserstoff oder C₁-C₈-Alkyl und R₂ C₁-C₈-Alkyl bedeuten.

Die erfindungsgemäss verwendeten Hydrierkatalysatoren sind Pt, Pd, Pt/Pd oder Rh auf einem Träger. Als Träger kommen die in der Technik der Hydrierkatalysatoren üblichen in Frage, beispielsweise Kohle (z.B. Aktivkohle, Holzkohle, Torfkohle), Kieselgur, Aluminiumoxid, Bariumsulfat usw. Bevorzugt als Träger ist Kohle. Bevorzugte erfindungsgemässe Katalysatoren sind Pt-, Pd- oder Pt/Pd-, insbesondere Pt- auf Kohle-Katalysatoren.

Die Menge an Edelmetall auf dem Träger (Belegung) bewegt sich in den für Hydrierkatalysatoren üblichen Bereichen. Sie beträgt z.B. 0,1 bis 10 %, beispielsweise 0,5 bis 10 %, vorzugsweise 1 bis 10 %, insbesondere 3 bis 10 %. Besonders zweckmässig sind Belegungen von 3 bis 7 %, z.B. etwa um 5 %, jeweils bezogen auf das Gewicht des Trägermaterials.

Der Katalysator wird zweckmässig in einer Menge von 0,1 - 6 %, insbesondere 0,5 - 4 %, beispielsweise 1,0 - 3,0 %, bezogen auf die eingesetzte o-Nitroazoverbindung, verwendet. Der Katalysator ist selbstverständlich recyclierbar, zweckmässig durch Filtration, wenn das Verfahren batchwise (diskontinuierlich) durchgeführt wird.

Als Alkylendiamine kommen insbesondere solche mit 1 bis 8, vorzugsweise 1 bis 6, vor allem 2 bis 8, z.B. 2 bis 6, C-Atomen in Betracht, wobei die Alkylengruppe geradkettig oder verzweigt sein kann. Die Aminogruppen der Alkylendiamine können durch C₁-C₆-, vorzugsweise C₁-C₄-Alkylgruppen substituiert sein. Die Alkylendiamine können somit durch maximal 4 Alkylgruppen substituiert sein bevorzugt ist im Molekül jedoch mindestens eine NH- oder NH₂-Gruppe vorhanden. Bevorzugte Alkylsubstituenten sind Methylgruppen oder Ethylgruppen. Beispiele hiefür sind Ethylendiamin, n-Propylendiamin, n-Butlyendiamin, n-Pentylendiamin, n-Hexylendiamin, 1-Amino-2-methylaminoethan, 1-Amino-3-methylaminopropan, 1-Amino-4-methylaminobutan, 1-Amino-3-dimethylaminopropan, 1-Amino-3-diethylaminopropan usw.. Acyclische Polyalkylenpolyamine sind beispielsweise solche mit 3 bis 6 Aminfunktionen und 2 bis 28, beispielsweise 2 bis 20, vorzugsweise 4 bis 18, insbesondere 4-12 C-Atomen. Zweckmässige Polyalkylenpolyamine entsprechen der Formel (Rₒ)₂N-CₙH₂ₙ(NRₒ-CₘH₂ₘ)ₚ-N(Rₒ)₂, worin p eine Zahl von 0 bis 4 und m und n unabhängig voneinander eine Zahl von 1 bis 6, insbesondere 1 bis 4, bedeuten, wobei im Fall p>1 die einzelnen Indices m gleich oder verschieden sind, und die Rₒ gleich oder verschieden sind und Wasserstoff oder C₁-C₆-, insbesondere C₁-C₄-Alkyl, vor allem Methyl oder Ethyl, bedeuten. Wasserstoff als Bedeutung von Rₒ ist bevorzugt. Bevorzugt handelt es sich bei den Alkylenketten um unverzweigte Reste. Die Summe aller n + m ist vorzugsweise 2-20, insbesondere 4-18, z.B. 4-12. Der Index p ist vorzugsweise eine Zahl von 1 bis 3, vor allem 1 oder 2. Im Fall der Alkylsubstitution von Aminogruppen durch Alkylgruppen können alle Aminogruppen durch die genannten Alkylgruppen substituiert sein. Bevorzugt ist im Molekül jedoch mindestens eine -NH- oder -NH₂-Gruppe vorhanden. Bevorzugt enthält ein Polyalkylenpolyamin 1 bis 3, z.B. 1 oder 2, insbesondere einen Alkylsubstituenten.

Besonders zweckmässige Polyalkylenpolyamine entsprechen der Formel (Rₒ)₂N-(CH₂)_{n'}-NRₒ-(CH₂)_{m'}-[NRₒ-(CH₂)_{p'}]_{q}-N(Rₒ)₂, worin n', m' und p' unabhängig voneinander 2 bis 4 und q 0, 1 oder 2, insbesondere 0 oder 1, bedeuten.

Beispiele für Polyalkenpolyamine sind Diethylentriamin, Triethylentetramin, Di-n- oder -i-propylentriamin, Tri-n- oder -i-propylentetramin, Di-n-butylentriamin und Tri-n-butylentetramin, H₂N(CH₂)₃N(CH₃)(CH₂)₃NH₂, H₂N(CH₂)₂N(CH₃)(CH₂)₂NH₂, H₂N(CH₂)₂N(CH₃)(CH₂)₂NH(CH₃)(CH₂)₂NH₂, CH₃NH(CH₂)₂NH(CH₂)₂NHCH₃ usw.

Als cyclische Polyalkylenpolyamine sind z.B. gesättigte, mindestens 2 Stickstoffatome enthaltende ein- oder mehrkernige heterocyclische Ringe zu nennen. Die einzelnen Heterocyclen sind insbesondere 5- bis 7-gliedrig, z.B. 6-gliedrig. Beispiele für einkernige cyclische Polyalkylenpolyamine sind Imidazolidin, Pyrazolidin, Hexahydropyrimidin, Hexahydropyrazin und Piperazin. Von den mehrkernigen cyclischen Polyalkylenpolyaminen seien beispielsweise Triethylendiamin (Diazabicyclooctan), Diazabicyclononan, Diazabicycloundecan und Hexamethylentetramin (Urotropin) genannt. Soweit die genannten cyclischen Amine NH-Gruppen enthalten, können diese ebenfalls mit C₁-C₆-, insbesondere C₁-C₄-Alkylgruppen, vorzugsweise Methylgruppen, substituiert sein. Beispiele hiefür sind N-Methylpiperazin und N,N-Dimethylpiperazin.

Zweckmässig werden im erfindungsgemässen Verfahren als Amine gegebenenfalls an einem oder mehreren Stickstoffatomen mit Methylgruppen oder Ethylgruppen substituierte C₁-C₈-Alkylendiamine oder Polyalkylenpolyamine mit 3 bis 6 Aminfunktionen und 2 bis 28, insbesondere 2 bis 20, C-Atomen, Piperazin, N-Methylpiperazin, Triethylendiamin oder Hexamethylentetramin eingesetzt. Bevorzugte Amine sind C₂-C₆-Alkylendiamine oder Polyalkylenpolyamine der Formel H₂N-(CH₂)_{n'}NH-(CH₂)_{m'}-[NH-(CH₂)_{p'}]_{q}-NH₂, worin die allgemeinen Symbole die vorstehend angegebenen Bedeutungen haben und wobei ein oder mehrere Stickstoffatome mit Methylgruppen oder Ethylgruppen substituiert sein können, oder Piperazin, N-Methylpiperazin oder Triethylendiamin. Besonders bevorzugte Amine sind Ethylendiamin, n-Propylendiamin, 1-Amino-3-dimethylamino-n-propan, 1-Amino-3-diethylamino-n-propan, n-Butylendiamin, n-Pentylendiamin, n-Hexylendiamin, Diethylentriamin, Triethylentetramin, Di-n-propylenmamin, Tri-n-propylentetramin, Di-n-butylentriamin oder Tri-n-butylentetramin, 1-Amino-2-methylaminoethan, 1-Amino-3-methylaminopropan, H₂N(CH₂)₃N(CH₃)(CH₂)₃NH₂, Piperazin, N-Methylpiperazin oder Triethylendiamin, vor allem Ethylendiamin, n-Propylendiamin, Diethylenmamin, Triethylentetramin oder Piperazin, insbesondere Ethylendiamin.

Es können selbstverständlich auch Mischungen aus zwei oder mehreren der genannten Amine im erfindungsgemässen Verfahren eingesetzt werden.

Das organische Amin ist im Reaktionsgemisch zwewckmässig in einer Menge von mindestens 0,01 Mol, insbesondere mindestens 0,1 Mol, vorzugsweise mindestens 0,4 Mol, bis etwa 8 Mol pro Mol o-Nitroazobenzol-Ausgangsprodukt vorhanden. Besonders zweckmässig sind Molverhältnisse Amin:Azoverbindung im Bereich von etwa 0,5:1 bis 1:1, insbesondere etwa 1:1.

Als Lösungsmittel fungiert im erfindungsgemässen Verfahren (wenn R₃ ≠ H) ein aromatischer Kohlenwasserstoff (z.B. Benzol oder alkylsubstituierte Benzole) oder halogenierte aromatische Kohenwasserstoffe (z.B. chlorierte Benzole wie etwa Chlorbenzol, Dichlorbenzol und Trichlorbenzol). Aromatische Kohlenwaserstoffe sind bevorzugt, insbesondere Benzol, Toluol oder Xylole. Es können auch Mischungen der genannten Lösungsmittel verwendet werden. Werden halogenierte Kohlenwasserstoffe als Lösungsmittel eingesetzt, wird vorzugsweise unter milden Hydrierbedingungen gearbeitet. Im erfindungsgemässen Verfahren stört auch die Anwesenheit von Wasser nicht, z.B. in Mengen bis zu 50 %, z.B. bis zu 30 %, bezogen auf das Gesamtlösungsmittel. Das erfindungsgemässe Verfahren wird jedoch in Abwesenheit von mit Wasser mischbaren organischen Lösungsmitteln (z.B. Alkoholen) durchgeführt.

Wird eine Azoverbindung der Formel II mit R₂ = CₙH₂ₙ-COOH als Ausgangsprodukt eingesetzt, wird die Hydrierung in Wasser oder einem Gemisch aus Wasser und einem gegebenenfalls halogenierten aromatischen Kohlenwasserstoff als Lösungsmittel durchgeführt. Bevorzugte Kohlenwasserstoffe sind vorstehend genannt. Das Lösungsmittelsystem enthält in diesem Fall zweckmässig die zur Lösung des Endproduktes nötige Menge an Wasser (um die Abtrennung des Katalysators, z.B. durch Filtration, zu ermöglichen), vorzugsweise mindestens 30 %, insbesondere mindestens 50 %, vor allem mindestens 70 % Wasser.

Das erfindungsgemässe Verfahren kann diskontinuierlich (batchwise), aber auch kontinuierlich durchgeführt werden. Für die kontinuierliche Verfahrensweise eignet sich insbesondere ein Festbett-Katalysator, z.B. eine Hochdruck-Festbetthydrieranlage. Das Reaktionsgemisch wird in diesem Fall kontinuierlich abgezogen und mit frischer Nitroazoverbindung + Amin + Lösungsmittel gespeist.

Eine besonders vorteilhafte Variante des erfindungsgemässen Verfahrens, die eine kontinuierliche Arbeitsweise erlaubt und zu hohen Umsätzen und kurzen Reaktionszeiten führt, besteht darin, dass man in einem Autoklaven den Katalysator in einem Teil des Lösungsmittels vorlegt, den Autoklaven unter Wasserstoffdruck setzt und dann die entsprechende Verbindung der Formel II, gelöst oder dispergiert in einem weiteren Teil des Lösungsmittels, zudosiert, z.B. mit Hilfe einer Dosierpumpe. Die Reaktionslösung kann dann kontinuierlich abgezogen und daraus das Endprodukt in üblicher Weise isoliert werden. Alternativ kann auch in diskontinuierlicher Weise der Katalysator abfiltriert und das Filtrat entsprechend aufgearbeitet werden.

Die Hydrierung wird zweckmässig bei Temperaturen von 0 - 120°C, z.B. 15 - 100°C, insbesondere 20 - 80°C durchgeführt. Besonders vorteilhaft sind Reaktionstemperaturen von 25 - 70°C, insbesondere von 40 - 70°C, z.B. 50 - 60°C.

Der Wasserstoffdruck kann bei der Hydrierung beispielsweise im Bereich von 1 - 200, beispielsweise 1 - 100, insbesondere 5 - 50, vorzugsweise 10 - 20 bar liegen. Welcher Wasserstoffdruck angewendet wird, hängt hauptsächlich von der zur Verfügung stehenden Hydrieranlage ab. Bei Hochdruckanlagen sind Drucke von 100 - 200 bar möglich. Solche sind vor allem bei kontinuierlicher Arbeitsweise üblich.

Die Hydrierzeit kann in weiten Grenzen schwanken, sie ist abhängig vom verwendeten Katalysator, vom Wasserstoffdruck, von der Reaktionstemperatur und der verwendeten Anlage. Sie kann z.B. von 30 Sekunden bis 5 Stunden betragen, insbesondere 10 Minuten bis 3 Stunden, z.B. 10 Minuten bis 2 Stunden. Bei kontinuierlicher Arbeitsweise ist z.B. in der Praxis mit Verweilzeiten von 1 bis 60 Minuten, insbesondere von 1 bis 30 Minuten zu rechnen.

Die Isolierung der Endprodukte aus dem Reaktionsmedium erfolgt nach üblichen, dem Fachmann bekannten Methoden. Sie variiert je nach der Art des verwendeten Lösungsmittels. Eine zweckmässige Methode besteht in der Ausfällung aus dem gegebenenfalls vorher eingeengten Reaktionsgemisch durch Zugabe eines Lösungsmittels, in dem das jeweilige Endprodukt schwer löslich ist und durch Abfiltrieren des Niederschlages. Aufarbeitung und allfällige Reinigungsoperationen sind auch den Beispielen zu entnehmen.

Wie bereits eingangs erwähnt, stellen die erfindungsgemäss herstellbaren 2-(2-Hydroxyphenyl)-2H-benztriazole wertvolle UV-Absorber dar, die in der Praxis als Lichtschutzmittel für eine Vielzahl von Anwendungen (wie beispielsweise in der Einleitung aufgezählt) eingesetzt werden können. Detaillierte Verwendungsmöglichkeiten der genannten Benztriazole sind in den US-A 3,055,896, 3,004,896, 3,072,585, 3,074,910, 3,189,615 und 3,230,194 beschrieben. Das erfindungsgemässe Verfahren eröffnet einen technisch besonders günstigen und wirtschaftlichen Weg zu deren Herstellung.

Die nachfolgenden Beispiele erläutern das erfindungsgemässe Verfahren weiter. Darin sowie in der übrigen Beschreibung und den Patentansprüchen bedeuten Teile Gewichtsteile und Prozentangaben Gewichtsprozent, sofern nichts anderes angegeben ist.

### Beispiel 1: 2-(2'-Hydroxy-5'-methylphenyl)-2H-benztriazol

In einen 300 ml Hydrierteaktor gibt man bei Raumtemperatur unter Argon 60 g 2-Nitro-2'-hydroxy-5'-methylazobenzol (Reinheit 91 %), 1,2 g 5 % Pt auf Aktivkohle, 80 g Xylol und 20 g Diethylentriamin. Anschliessend wird Argon durch Wasserstoff ersetzt. Nach Aufdrücken von 10 bar Wasserstoff wird unter intensiver Rührung bei 60°C hydriert. Durch Kühlung wird die freiwerdende Wärme abgeführt. Das Ende der Hydrierreaktion ist durch den Stillstand der Wasserstoffaufnahme nach 2 Moläquivalenten Wasserstoff, bezogen auf das Azobenzol-Ausgangsprodukt, leicht zu erkennen. Die gesamte Hydrierzeit beträgt ca. 0,7 Stunden.

Nach Erhitzen der Reaktionsmasse auf 80°C wird der Katalysator abfiltriert. Die Reaktionslösung besteht aus einer schwarzen, aminreichen Unterphase und einer gelben bis leicht bräunlichen, das Produkt enthaltenden xylolischen Oberphase. Die aminreiche Unterphase wird zur besseren Aufarbeitung und Wiederverwendung des Amins abgetrennt.

Durch Zugabe von 20 g Wasser (bei 70 - 75°C) zur das Produkt enthaltenden Phase und anschliessender Abtrennung der wässrig-aminischen Phase wird das Diethylenmamin praktisch quantitativ aus der Xylolphase extrahiert. Anschliessend wird die verbleibende xylolische Produktphase durch Destillation unter Vakuum aufkonzentriert. Durch Zugabe von 160 g Methanol (beginnend bei ca. 90°C unter anschliessender Siedekühlung) wird das Produkt ausgefällt. Nach Abkühlung der Kristalldispersion auf 0°C filtriert man die Kristalle und trocknet sie. Ausbeute 44,0 g der Titelverbindung, entsprechend 92 % der Theorie. Schmelzpunkt: 128°C.

### Beispiele 2-4:

Beispiel 1 wird wiederholt, wobei jedoch anstelle von 80 g Xylol und 20 g Diethylentriamin 30 g Toluol und 70 g Diethylentriamin eingesetzt werden. Es ist kein Einfluss auf die Ausbeute zu beobachten. Ebenso zeigt ein Ersatz von Diethylentriamin durch Triethylentetramin oder durch Ethylendiamin keinen negativen Effekt. Man erhält jeweils praktisch die gleiche Ausbeute wie in Beispiel 1 angegeben.

### Beispiele 5-8:

Die Beispiele 1 bis 4 werden wiederholt, wobei jedoch der Hydrierdruck von 10 auf 80 bar Wasserstoff erhöht wird. Die Hydrierzeit wird dadurch von 0,7 Stunden auf 0,3 Stunden verkürzt. Die Aufarbeitung ist analog, man erhält jeweils praktisch die gleiche Ausbeute.

### Beispiele 9-13:

Beispiel 1 wird wiederholt, wobei jedoch anstelle des 5 % Pt auf Aktivkohle-Katalysators die gleiche Menge eines 1 %, 2 %, 3 %, 4 % bzw. 10 % Pt auf Aktivkohle-Katalystors eingesetzt wird. Die Hydrierzeit nimmt mit steigender Pt-Belegung des Katalysators stetig ab. Sie beträgt für den 1 % Pt-Katalysator ca. 3 Stunden und für den 10 % Pt-Katalysator ca. 0,5 Stunden. Die Aufarbeitung ist analog. Die erhaltene Produktausbeute wird durch die unterschiedliche Edelmetallbelegung des Katalysators kaum beeinflusst. Man isoliert das Produkt in einer Ausbeute von 90 ± 2 % der Theorie.

### Beispiele 14 und 15:

Beispiel 1 wird wiederholt, wobei jedoch die Hydriertemperatur von 60°C auf 30°C erniedrigt bzw. auf 80°C erhöht wird. Ausser einer Beeinflussung der Hydrierzeit (Verlängerung auf ca. 5 Stunden im ersten Fall bzw. Verkürzung auf ca. 0,5 Stunden im zweiten Fall) wird kein signifikanter Effekt beobachtet. Man isoliert das Produkt in ähnlicher Ausbeute wie in Beispiel 1.

### Beispiel 16:

In einen 2 l-Hydrierreaktor gibt man bei Raumtemperatur unter Argon 100 g Diethylentriamin, 100 g Xylol und 6 g 5 % Pt auf Aktivkohle. Der Reaktor wird verschlossen und Argon durch Wasserstoff ersetzt. Nach Aufdrücken von 10 bar Wasserstoff wird der Katalysator durch intensive Rührung dispergiert. Gleichzeitig werden in einem externen Behälter 300 g 2-Nitro-2'-hydroxy-5'-methylazobenzol (Reinheit 91 %) in 300 g Xylol und 40 g Wasser bei Raumtemperatur dispergiert. Diese Dispersion wird mit einer automatischen Dosiereinrichtung über einen Zeitraum von einer Stunde gegen den Wasserstoffdruck in den Hydrierteaktor gepumpt. Dabei findet die Hydrierung der Azoverbindung bei 60°C zu dem entsprechenden Benztriazol statt.

Nach Beendigung der Dosierung wird die Reaktionsmasse auf 80°C aufgeheizt und der Katalysator abfiltriert. Die weitere Aufarbeitung erfolgt analog Beispiel 1 mit den entsprechend erhöhten Lösungsmittelmengen. Man erhält 221 g 2-(2'-Hydroxy-5'-methylphenyl)-2H-benztriazol entsprechend 92,5 % der Theorie. Schmelzpunkt: 128°C.

### Beispiele 17-19:

Beispiel 1 wird wiederholt, wobei jedoch anstelle des 5 % Pt auf Aktivkohle-Katalysators die gleiche Menge eines Pd/Pt Mischkatalysators (4 % Pd/1 % Pt auf Aktivkohle) bzw. eines 5 % Pd auf Aktivkohle-Katalysators eingesetzt werden. Es ist praktisch kein Einfluss auf die Hydrierzeit zu beobachten. Das Produkt wird analog Beispiel 1 in einer Ausbeute von 90 % der Theorie isoliert.

Beim Einsatz eines 5 % Rh auf Aktivkohle-Katalysators isoliert man nach gleicher Arbeitsweise das Produkt in einer Ausbeute von 88 % der Theorie.

### Beispiel 20:

Beispiel 1 wird wiederholt, wobei jedoch zusätzlich zur Hydrierlösung noch 8 g Wasser zugefügt werden. Es ist kein Effekt auf die Ausbeute oder Hydrierzeit zu beobachten. Das Produkt wird in einer Ausbeute von 92% der Theorie isoliert.

### Beispiel 21: 2-(2'-Hydroxy-5'-tert.-octylphenyl)-2H-benztriazol

Beispiel 1 wird wiederholt, wobei jedoch anstelle des 2-Nitro-2'-hydroxy-5'-methylazobenzols eine äquivalente Menge an 2-Nitro-2'-hydroxy-5'-tert.-octylazobenzol (Reinheit 93 %) und anstelle von 80 g Xylol und 20 g Diethylenmamin eine Mischung aus 60 g Xylol und 40 g Diethylenmamin eingesetzt wird. Die Hydrierung wird bei 45°C durchgeführt. Das Titelprodukt wird in einer Ausbeute von 92 % der Theorie isoliert. Schmelzpunkt: 101-103°C.

### Beispiel 22: 2-(2'-Hydroxy-3',5'-di-tert.-butylphenyl)-2H-benztriazol

Beispiel 1 wird wiederholt, wobei jedoch anstelle des 2-Nitro-2'-hydroxy-5'-methylazobenzols eine äquivalente Menge an 2-Nitro-2'-hydroxy-3',5'-di-tert.-butylazobenzols (Reinheit 91 %) und anstelle von 80 g Xylol und 20 g Diethylenmamin eine Mischung aus 40 g Xylol und 60 g Diethylenmamin eingesetzt werden. Die Hydrierung wird bei 50°C durchgeführt. Nach Aufarbeitung der Hydrierlösung wird das Produkt aus einer Mischung von 40 g Xylol und 160 g Methanol kristallisiert. Das Produkt (Titelverbindung) wird in einer Ausbeute von 90 % der Theorie isoliert. Schmelzpunkt: 152-154°C.

### Beispiel 23: 2-(2'-Hydroxy-3'-isobutyl-5'-tert.-butylphenyl)-2H-benztriazol

Beispiel 22 wird wiederholt, wobei jedoch anstelle der dort eingesetzten Azoverbindung die gleiche Menge 2-Nitro-2'-hydroxy-3'-isobutyl-5'-tert.-butylazobenzol (Reinheit 86 %) verwendet wird.

Die Hydrierung wird bei 40 - 50°C durchgeführt; die Hydrierzeit beträgt ca. 0,7 Stunden. Nach der Katalysatorfiltration und der Abtrennung der aminischen Unterphase wird die verbleibende Reaktionslösung 2 mal (bei 70 - 75°C) mit je 20 g Wasser extrahiert. Dadurch wird das Amin praktisch quantitativ aus der Reaktionslösung entfernt.

Nach Abdestillation des Xylols wird die verbleibende Produktschmelze bei 60 - 70°C mit 160 g Methanol versetzt. Durch langsames Abkühlen und Animpfen bei 50 - 60°C wird das Produkt (Titelverbindung) auskristallisiert. Nach Abkühlen auf 0°C und Filtration sowie Trocknung wird die Titelverbindung in einer Ausbeute von 87 % der Theorie erhalten. Schmelzpunkt: 79-80°C.

### Beispiel 24: 2-(2'-Hydroxy-3',5'-di-tert.-amylphenyl)-2H-benztriazol

Beispiel 1 wird wiederholt, wobei jedoch anstelle des 2-Nitro-2'-hydroxy-5'-methylazobenzols eine äquivalente Menge an 2-Nitro-2'-hydroxy-3',5'-di-tert.-amylazobenzol (Reinheit 91,6 %) sowie anstelle von 80 g Xylol und 20 g Diethylentriamin eine Mischung aus 60 g Xylol und 40 g Diethylentriamin eingesetzt wird. Die Aufarbeitung ist analog (Filtration des Katalysators, Abtrennen der aminischen Unterphase, Extraktion des restlichen Amins aus der xylolischen Oberphase mit Wasser, Aufkonzentrierung und Kristallisation aus Xylol/Methanol). Das Titelprodukt wird in einer Ausbeute von 86,5 % der Theorie isoliert. Schmelzpunkt: 80-82°C.

### Beispiel 25: 2-(2'-Hydroxy-3',5'-bis-α,α-dimethylbenzylphenyl)-2H-benztriazol

Beispiel 22 wird wiederholt, wobei jedoch anstelle der dort verwendeten Azoverbindung eine äquivalente Menge an 2-Nitro-2'-hydroxy-3',5'-bis-α,α-dimethylbenzylazobenzol (Reinheit 90,3 %) eingesetzt wird.

Die Hydriertempertur beträgt statt 50°C 40°C. Nach der Aufarbeitung der Hydrierlösung wird das Produkt aus Xylol/Methanol (Verhältnis: Produkt/ Xylol/Methanol = 1 : 1 : 3) auskristallisiert. Das Titelprodukt wird in einer Ausbeute von 92,5 % der Theorie isoliert. Schmelzpunkt: 137-138°C.

### Beispiel 26: 2-[2'-Hydroxy-3'-tert.-butyl-5'-(2˝-methoxycarbonylethyl)-phenyl]-2H-benztriazol

Beispiel 1 wird wiederholt, wobei jedoch anstelle der dort verwendeten Azoverbindung eine äquivalente Menge 2-Nitro-2'-hydroxy-3'-tert.-butyl-5'-(2˝-methoxycarbonylethyl)azobenzol (Reinheit 99,4 %) und anstelle des 5 % Pt auf Aktivkohle-Katalysators die gleiche Menge eines 1 % Pt auf Aktivkohle-Katalysators verwendet werden. Es wird bei 40°C hydriert, die Hydrierzeit beträgt dabei ca. 1,2 Stunden.

Nach der Abtrennung des Katalysators und des Amins wird die xylolische Produktphase aufkonzentriert und das Produkt (Titelverbindung) durch Zugabe von Methanol ausgefällt (Verhältnis Produkt:Xylol:Methanol ca. 1:1,5:4). Die Kristalle werden abgetrennt und getrocknet. Die so erhaltene Titelverbindung weist einen Schmelzpunkt von 125-126°C auf.

### Beispiele 27 und 28: 2-(2'Hydroxy-5'-methylphenyl)-2H-benztriazol

Beispiel 1 wird wiederholt, wobei jedoch anstelle von 20 g Diethylenmamin und 80 g Xylol eine Mischung aus 40 g Piperazinhexahydrat und 60 g Xylol oder aus 18 g Piperazin und 60 g Xylol eingesetzt wird.

Das Produkt wird analog Beispiel 1 in beiden Fällen in einer Ausbeute von 91 % der Theorie isoliert (Schmelzpunkt 127-128°C).

### Beispiele 29 und 30:

Beispiel 1 wird wiederholt, wobei jedoch anstelle des Diethylentriamins 22 g 1-Amino-3-dimethylamino-n-propan oder 27 g 1-Amino-3-diethylamino-n-propan eingesetzt werden. Das Titelprodukt wird im ersten Fall mit einer Ausbeute von 88 %, im zweiten Fall mit einer Ausbeute von 86 % der Theorie erhalten. Schmelzpunkt: 128°C.

## Patentansprüche

1. Verfahren zur Herstellung von 2-(2-Hydroxyphenyl)-2H-benztriazolen der Formel 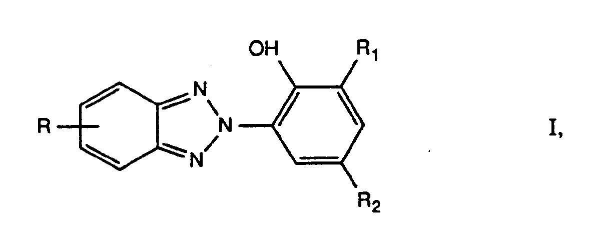 worin R Wasserstoff, C₁-C₁₂-Alkyl oder C₁-C₄-Alkoxy, R₁ Wasserstoff, C₁-C₁₂-Alkyl, C₅-C₆-Cycloalkyl, Phenyl oder Phenyl-C₁-C₄-alkyl und R₂ C₁-C₁₂-Alkyl, C₅-C₆-Cycloalkyl, Phenyl, Phenyl-C₁-C₄-alkyl oder eine Gruppe -CₙH₂ₙ-COOR₃ bedeuten, worin n für 0 bis 4 und R₃ für Wasserstoff oder C₁-C₁₂-Alkyl stehen, durch katalytische Hydrierung einer Azoverbindung der Formel 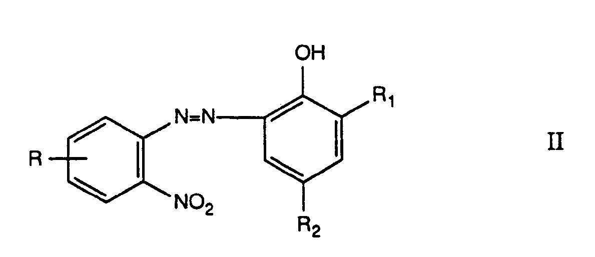 in Gegenwart eines Edelmetall-Hydrierkatalysators und eines organischen Amins, dadurch gekennzeichnet, dass man als Hydrierkatalysator Pt, Pd, Pt/Pd oder Rh auf einem Träger und als Amin ein Alkylendiamin oder ein acyclisches oder cyclisches Polyalkylenpolyamin einsetzt, wobei die Stickstoffatome der genannten Amine unsubstituiert oder, unabhängig voneinander, mit C₁-C₆-Alkyl substituiert sind, und die Hydrierung in einem aromatischen Kohlenwasserstoff oder halogenierten aromatischen Kohlenwasserstoff oder Mischungen der genannten Kohlenwasserstoffe mit Wasser als Lösungsmittel durchführt, wobei man die Hydrierung von Azoverbindungen der Formel II, worin R₂ = -CₙH₂ₙCOOH in Wasser oder in Mischungen der genannten Kohlenwasserstoffe und Wasser durchführt.

2. Verfahren nach Anspruch 1, worin R₁ Wasserstoff, C₁-C₁₂-Alkyl oder Phenyl-C₁-C₃-alkyl und R₂ C₁-C₁₂-Alkyl, Phenyl-C₁-C₃-alkyl oder eine Gruppe -C₂H₄COOR₃ bedeuten, worin R₃ H oder C₁-C₁₂-Alkyl, insbesondere H oder C₁-C₄-Alkyl, bedeutet.

3. Verfahren nach Anspruch 1, worin als Katalysator 1 - 10%, vorzugsweise 3-7 %, Rh, Pt, Pd oder Pt/Pd auf Kohle verwendet wird

4. Verfahren nach einem der Ansprüche 1-3, worin als organisches Amin ein gegebenenfalls an einem oder mehreren Stickstoffatomen mit Methylgruppen oder Ethylgruppen substituiertes C₁-C₈-Alkylendiamin oder Polyalkylenpolyamin mit 3 bis 6 Aminfunktionen und 2 bis 28, insbesondere 2 bis 20, C-Atomen, Piperazin, N-Methylpiperazin, Triethylendiamin oder Hexamethylentetramin eingesetzt wird

5. Verfahren nach Anspruch 4, worin das organische Amin ein C₂-C₈-Alkylendiamin oder ein Polyalkylenpolyamin der Formel H₂N-(CH₂)_{n'}-NH-(CH₂)_{m'}-[NH-(CH₂)_{p'}]_{q}-NH₂ ist, worin n', m' und p' unabhängig voneinander 2 bis 4 und q 0,1 oder 2, insbesondere 0 oder 1, bedeuten und das gegebenenfalls an einem oder mehreren Stickstoffatomen mit Methylgruppen oder Ethylgruppen substituiert ist, oder Piperazin, N-Methylpiperazin oder Triethylendiamin ist.

6. Verfahren nach Anspruch 4, worin das Amin Ethylendiamin, n-Propylendiamin, 1-Amin-3-dimethylamino-n-propan, 1-Amino-3-diethylamino-n-propan, n-Butylendiamin, n-Pentylendiamin, n-Hexylendiamin, Diethylentriamin, Triethylentetramin, Di-n-propylentriamin, Tri-n-propylentetramin, Di-n-butylentriamin, Tri-n-butylentetramin, 1-Amino-2-methylaminoethan, 1-Amino-3-methylaminopropan, H₂N(CH₂)₃N(CH₃)(CH₂)₃NH₂, Piperazin, N-Methylpiperazin oder Triethylendiamin ist.

7. Verfahren nach Anspruch 6, worin das Amin Ethylendiamin, n-Propylendiamin, Diethylenmamin, Triethylentetramin oder Piperazin ist.

8. Verfahren nach einem der Ansprüche 1-7, worin als Lösungsmittel ein aromatischer Kohlenwasserstoff eingesetzt wird.

9. Verfahren nach Anspruch 8, worin das Lösungsmittel Benzol, Toluol oder ein Xylol ist.

10. Verfahren nach Anspruch 1, worin im Fall R₂ = CₙH₂ₙ-COOH Wasser als Lösungsmittel verwendet wird

11. Verfahren nach Anspruch 1, worin das Amin in einer Menge von mindestens 0,01 Mol, insbesondere mindestens 0,5 Mol pro Mol Azoverbindung der Formel II eingesetzt wird.

## Claims

1. A process for the preparation of 2-(2-hydroxyphenyl)-2H-benzotriazoles of the formula 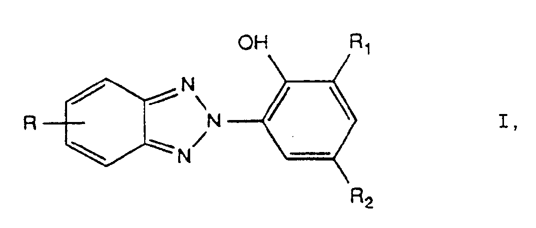 in which R is hydrogen, C₁-C₁₂alkyl or C₁-C₄alkoxy, R₁ is hydrogen, C₁-C₁₂alkyl, C₅-C₆cycloalkyl, phenyl or phenyl-C₁-C₄alkyl and R₂ is C₁-C₁₂alkyl, C₅-C₆cycloalkyl, phenyl, phenyl-C₁-C₄alkyl or a group -CₙH₂ₙ-COOR₃, in which n is O to 4 and R₃ is hydrogen or C₁-C₁₂alkyl, by catalytic hydrogenation of an azo compound of the formula 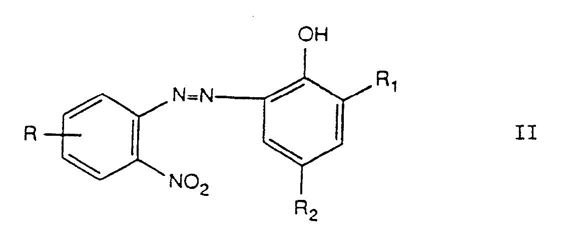 in the presence of a noble metal hydrogenation catalyst and an organic amine, which comprises using Pt, Pd, Pt/Pd or Rh on a support as hydrogenation catalyst and an alkylenediamine or an acyclic or cyclic polyalkylene polyamine as amine, in which the nitrogen atoms of the amines mentioned are unsubstituted or, independently of one another, substituted by C₁-C₆alkyl, and carrying out the hydrogenation in an aromatic hydrocarbon or halogenated aromatic hydrocarbon or mixtures of the hydrocarbons mentioned with water as solvent, which hydrogenation of azo compounds of the formula II, in which R₂ is -CₙH₂ₙCOOH, is carried out in water or in mixtures of the hydrocarbons mentioned and water.

2. A process according to claim 1, wherein R₁ is hydrogen, C₁-C₁₂alkyl or phenyl-C₁-C₃alkyl and R₂ is C₁-C₁₂alkyl, phenyl-C₁-C₃alkyl or a group -C₂H₄COOR₃, in which R₃ is H or C₁-C₁₂alkyl, in particular H or C₁-C₄alkyl.

3. A process according to claim 1, wherein 1-10 %, preferably 3-7 %, Rh, Pt, Pd or Pt/Pd on carbon is used as catalyst.

4. A process according to any one of claims 1-3, wherein the organic amine used is a C₁-C₈alkylenediamine which is unsubstituted or substituted on one or more nitrogen atoms by methyl groups or ethyl groups, or is a polyalkylene polyamine having 3 to 6 amine functions and 2 to 28, in particular 2 to 20, C atoms, or is piperazine, N-methylpiperazine, triethylenediamine or hexamethylenetetramine.

5. A process according to claim 4, wherein the organic amine is a C₂-C₈alkylenediamine or a polyalkylene polyamine of the formula H₂N-(CH₂)ₙ,-NH-(CH₂)ₘ,-[NH-(CH₂)ₚ,]_{q}-NH₂, in which n', m' and p', independently of one another, are 2 to 4, and q is 0, 1 or 2, in particular 0 or 1, and which is unsubstituted or substituted on one or more nitrogen atoms by methyl groups or ethyl groups, or is piperazine, N-methylpiperazine or triethylenediamine.

6. A process according to claim 4, wherein the amine is ethylenediamine, n-propylenediamine, 1-amino-3-dimethylamino-n-propane, 1-amino-3-diethylamino-n-propane, n-butylenediamine, n-pentylenediamine, n-hexylenediamine, diethylenetriamine, triethylenetetramine, di-n-propylenetriamine, tri-n-propylenetetramine, di-n-butylenetriamine, tri-n-butylenetetramine, 1-amino-2-methylaminoethane, 1-amino-3-methylaminopropane, H₂N(CH₂)₃N(CH₃)(CH₂)₃NH₂, piperazine, N-methylpiperazine or triethylenediamine.

7. A process according to claim 6, wherein the amine is ethylenediamine, n-propylenediamine, diethylenetriamine, triethylenetetramine or piperazine.

8. A process according to any one of claims 1-7, wherein the solvent used is an aromatic hydrocarbon.

9. A process according to claim 8, wherein the solvent is benzene, toluene or a xylene.

10. A process according to claim 1, wherein, in the case that R₂ is CₙH₂ₙ-COOH, water is used as solvent.

11. A process according to claim 1, wherein the amine is used in an amount of at least 0.01 mole, in particular at least 0.5 mole, per mole of azo compound of the formula II.

## Revendications

1. Procédé de préparation de 2-(2-hydroxyphenyl)-2H-benzotriazoles de formule 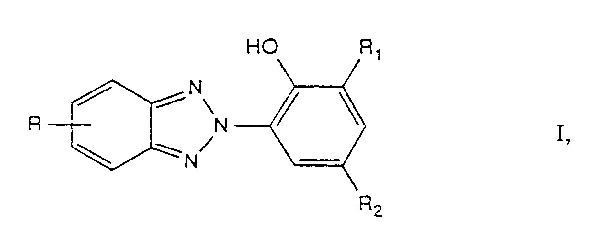 dans laquelle R représente un hydrogène, un alkyle en C₁-C₁₂ ou un alcoxy en C₁-C₄, R₁ représente un hydrogène, un alkyle en C₁-C₁₂, un cycloalkyle en C₅-C₆, un phényle ou un phényl-alkyle en C₁-C₄, et R₂ représente un alkyle en C₁-C₁₂, un cycloalkyle en C₅-C₆, un phényle, un phényl-alkyle en C₁-C₄ ou un groupe -CₙH₂ₙ-COOR₃, où n est 0 à 4 et R₃ est un hydrogène ou un alkyle en C₁-C₁₂, par hydrogénation catalytique d'un composé azoïque de formule 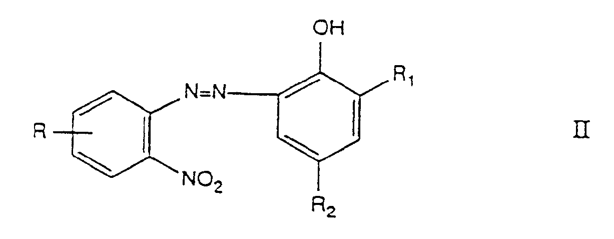 en présence d'un catalyseur d'hydrogénation à base d'un métal noble et d'une amine organique, caractérisé en ce que l'on utilise comme catalyseur d'hydrogénation Pt, Pd, Pt/Pd ou Rh sur un support, et comme amine une alkylènediamine ou une polyalkylènepolyamine acyclique ou cyclique, les atomes d'azote desdites amines étant non substitués ou, indépendamment l'un de l'autre, substitués par alkyle en C₁-C₆, et en ce que l'on effectue l'hydrogénation dans un hydrocarbure aromatique ou un hydrocarbure aromatique halogéné ou des mélanges desdits hydrocarbures avec de l'eau comme solvants, l'hydrogénation des composés azoïques de formule II dans laquelle R₂ = -CₙH₂ₙ-COOR₃ étant effectuée dans l'eau ou dans des mélanges desdits hydrocarbures et d'eau.

2. Procédé selon la revendication 1, dans lequel R₁ représente l'hydrogène, un alkyle en C₁-C₁₂ ou un phényl-alkyle en C₁-C₃ et R₂ est un alkyle en C₁-C₁₂, un phényl-alkyle en C₁-C₃ ou un groupe -C₂H₄-COOR₃, où R₃ signifie H ou alkyle en C₁-C₁₂, en particulier H ou alkyle en C₁-C₄.

3. Procédé selon la revendication 1, dans lequel on utilise comme catalyseur 1 - 10 %, de préférence 3 - 7 %, de Rh, Pt, Pd ou Pt/Pd sur du charbon.

4. Procédé selon l'une des revendications 1 - 3, dans lequel on utilise comme amine organique une alkylènediamine en C₁-C₈ ou une polyalkylènepolyamine ayant 3 à 6 fonctions amine et 2 à 28, en particulier 2 à 20 atomes de carbone, éventuellement substituées sur un ou plusieurs atomes d'azote par des groupes méthyle ou des groupes éthyle, la pipérazine, la N-méthylpipérazine, la triéthylènediamine ou l'hexaméthylènetétramine.

5. Procédé selon la revendication 4, dans lequel l'amine organique est une alkylènediamine en C₂-C₈ ou une polyalkylènepolyamine de formule H₂N-(CH₂)_{n'}-NH-(CH₂)_{m'}-[NH-(CH₂)_{p'}]_{q}-NH₂, dans laquelle n', m' et p' sont indépendamment l'un de l'autre compris entre 2 et 4 et q est 0, 1 ou 2, en particulier 0 ou 1, et qui peut être substituée sur un ou plusieurs atomes d'azote par des groupes méthyle ou des groupes éthyle, ou la pipérazine, la N-méthylpipérazine ou la triéthylènediamine.

6. Procédé selon la revendication 4, dans lequel l'amine est l'éthylènediamine, la n-propylènediamine, le 1-amino-3-diméthylamino-n-propane, le 1-amino-3-diéthylamino-n-propane, la n-butylènediamine, la n-pentylènediamine, la n-hexylènediamine, la diéthylènetriamine, la triéthylènetétramine, la di-n-propylènetriamine, la tri-n-propylènetétramine, la di-n-butylènetriamine, la tri-n-butylènetétramine, le 1-amino-2-méthylaminoéthane, le 1-amino-3-méthylaminopropane, H₂N-(CH₂)₃-N(CH₃)(CH₂)₃-NH₂, la pipérazine, la N-méthylpipérazine ou la triéthylènediamine.

7. Procédé selon la revendication 6, dans lequel l'amine est l'éthylènediamine, la n-propylènediamine, la diéthylènetriamine, la triéthylènetétramine ou la pipérazine.

8. Procédé selon l'une des revendications 1 - 7, dans lequel on utilise un hydrocarbure aromatique comme solvant.

9. Procédé selon la revendication 8, dans lequel le solvant est le benzène, le toluène ou un xylène.

10. Procédé selon la revendication 1, dans lequel, au cas où R₂ = -CₙH₂ₙ-COOH, on utilise de l'eau comme solvant.

11. Procédé selon la revendication 1, dans lequel on utilise l'amine en une quantité d'au moins 0,01 mole, en particulier d'au moins 0,5 mole par mole de composé azoïque de formule II.
